# FASCICULE DE BREVET EUROPEEN

(11) **EP 3 122 427 B1**
(45) Date de publication et mention de la délivrance du brevet: **25.07.2018**
(21) Numéro de dépôt: 15741782.5
(22) Date de dépôt: 24.03.2015
(51) Int. Cl.: A61Q 5/00, A61K 8/97

(54) **UTILISATION COSMETIQUE D'UN EXTRAIT DE MARGINES D'OLIVIER POUR REDENSIFIER LE CHEVEU**
KOSMETISCHE VERWENDUNG EINES EXTRAKTS AUS OLIVENBAUMPFLANZENWASSER ZUR ERHÖHUNG DER DICHTE VON HAAR
COSMETIC USE OF AN EXTRACT OF OLIVE-TREE VEGETABLE WATER TO INCREASE THE DENSITY OF HAIR

(30) Priorité: 24.03.2014 FR 1452431
(43) Date de publication de la demande: 01.02.2017
(73) Titulaire: Pierre Fabre Dermo-Cosmétique, 92100 Boulogne-Billancourt (FR)
(72) Inventeur: MANDEAU, Anne, F-31200 Toulouse (FR); BLACK, David, F-31860 Labarthe Sur Leze (FR)
(74) Mandataire: Regimbeau
(86) Numéro de dépôt international: PCT/EP2015/056180
(87) Numéro de publication internationale: WO 2015/144656

(56) Documents cités:
- JP-A- 2001 206 822
- US-A1- 2009 324 760

## Description

Le domaine de la présente invention concerne l'utilisation cosmétique d'un extrait de margines d'olivier pour son activité redensifiante du cheveu.

La production d'huile d'olive est assurée par des unités traditionnelles équipées de presses qui triturent les olives (moulins), et de plus en plus par des unités modernes discontinues ou continues, à deux ou trois phases, avec centrifugation. Les unités de trituration équipées de chaînes continues à trois phases associent deux centrifugations, la première pour séparer les grignons des huiles plus margines et la deuxième pour séparer les huiles des margines.

Les margines sont représentées par les eaux de végétation de l'olive et l'eau rajoutée au cours de l'extraction, les grignons étant représentés par la phase solide.

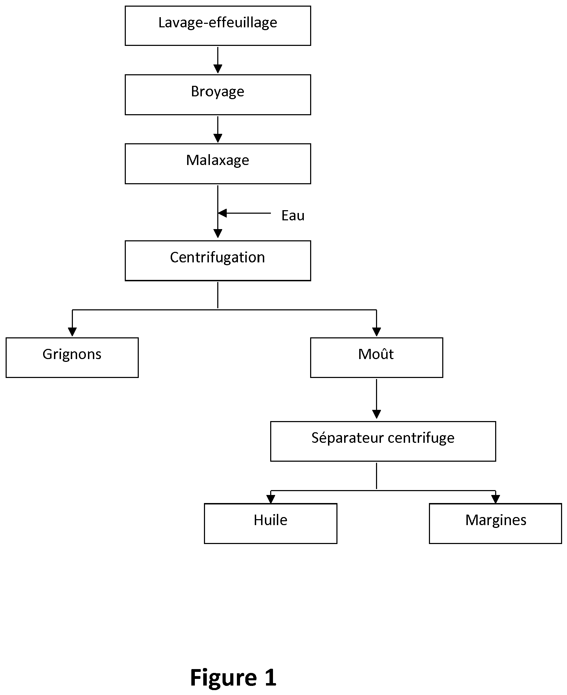

Le schéma de l'obtention d'huile d'olive par un système continu à trois phases est représenté à la figure 1 annexée.

Les rendements d'obtention de margines sont d'environ 90 L pour 100 kg d'olive, dans le cas d'un décanteur à trois phases.

Les margines peuvent également être récupérées en sortie d'un moulin à deux phases. Dans ce cas, les olives passent également dans un malaxeur, puis dans un séparateur solide / liquide qui sépare les grignons du mélange huile + eau. Ensuite, une ou plusieurs centrifugeuses séparent l'huile de l'eau. Dans un moulin à deux phases, il n'y a pas d'ajout d'eau au cours du procédé et le rendement est d'environ 50L de margines pour 100 kg d'olives.

Les margines issues des centrifugeuses sont des sous-produits à très forte charge polluante. Les concentrations en DCO (Demande Chimique en Oxygène) et DBO5 (Demande Biologique en Oxygène, mesurée après 5J) sont supérieures aux limites de rejets autorisés dans le réseau d'assainissement collectif. Les composés phénoliques présents dans les margines ont une action inhibitrice sur les populations de micro-organismes utilisés dans les stations d'épuration, et en font un sous-produit difficilement compostable, pour les mêmes raisons. Ces margines sont donc souvent entreposées dans des bassins d'évaporation, et non valorisées.

Cette forte teneur en composés phénoliques est pourtant très intéressante pour des applications pharmaceutiques et cosmétiques (Roig A, Cayuela ML and Sanchez-Monedero MA (2006) An overview on olive mill wastes and their valorisation methods. Waste Management 26:960-969).

Le profil phénolique ainsi que les propriétés anti-oxydantes des margines d'olives sont largement décrites dans la littérature (El-Abbassi A, Kiai H and Hafidi A (2012) Phenolic profile and antioxidant activities of olive mill wastewater. Food Chemistry, 132: 406-412).

Les composés phénoliques des olives possèdent également d'autres bénéfices santé tels que la prévention contre le cancer ou des activités neuroprotectrices. L'intérêt pour de nouvelles sources d'antioxydants alimentaires est grandissant. Or les margines récupèrent une grande majorité des composés phénoliques présents dans l'olive.

Les composés fondamentaux des margines sont l'eau (83,2%), les substances organiques (15 %) et les substances minérales (1,8 %). Les substances minérales sont composées d'azote, de phosphore, de potassium et de magnésium. Les substances organiques contiennent principalement des sucres (fructose, glucose, saccharose, etc.), des composés phénoliques et de l'huile résiduelle.

Des brevets de procédé et/ou d'utilisation ont été déposés sur ce sous-produit de la fabrication de l'huile d'olive.

Certains concernent l'utilisation de margines après filtration sur une membrane de 41000 Daltons (US200900053333), d'autres l'ajout d'acide pour stabiliser les margines et convertir l'oleuropéine en hydroxytyrosol (US20100216874), un procédé visant à purifier les composés phénoliques de faible poids moléculaire à partir de margines, associant clarification et nanofiltration (WO201094860), suivi parfois d'une osmose inverse (EP1910257), ou l'association microfiltration, ultra-filtration, nanofiltration et osmose inverse et enfin oxydation pour récupérer un maximum de tyrosol et hydroxytyrosol (EP1623960, WO2005123603). D'autres procédés comportent une extraction des margines avec un solvant polaire, une inactivation enzymatique par chauffage, une séparation solide / liquide et une concentration par séparation membranaire afin d'obtenir un extrait comprenant plus de 10% (w/w) de polyphénols (WO2006005986).

En outre, il existe des produits sur le marché contenant des margines d'olives pour leurs propriétés anti-oxydantes, en compléments alimentaires et en cosmétique.

Un brevet japonais divulgue le développement d'un shampoing contenant des margines associés entre autres à une silicone non volatile pour avoir un effet conditionneur sans entrainer de raideur des cheveux (JP2004051602). De même, un usage cosmétique pour la peau à base de margines est décrit dans le brevet JP2000319161. Le document JP2001206822 décrit que les compositions à base de margines d'olivier confèrent de la brillance et un caractère soyeux aux cheveux. Le document US2009/0324760 décrit que les compositions à base de margines d'olivier sont utilisées pour favoriser la pousse des cheveux. L'association entre un extrait de feuilles d'olivier et un extrait obtenu par traitement avec un solvant organique polaire des margines est décrite pour leur action contre les signes cutanés du vieillissement (application topique cutanée) dans le brevet FR2864785.

Enfin, l'activité antioxydante et cytoprotective d'une glucurolactone présente dans les margines est décrite pour son utilisation dans des produits pharmaceutiques et cosmétiques (GR2002100071).

La présente invention vise à fournir une nouvelle composition capillaire destinée à redensifier le cheveu.

Les travaux réalisés par la Demanderesse ont permis de mettre en évidence qu'un extrait de margines d'olives avait de bonnes propriétés épaississantes du cheveu.

La présente invention concerne l'utilisation d'une composition capillaire comprenant à titre de principe actif un extrait de margines d'olivier et comprenant en outre au moins un excipient cosmétiquement acceptable, pour redensifier le cheveu.

Dans le cadre de la présente invention, par « densité », « densifier », on entend une amélioration de la densité capillaire via une action épaississante du cheveu et non une action sur le nombre de cheveux par unité de surface.

Conformément à la présente invention, une augmentation de l'épaisseur du cheveu se traduit par une augmentation de sa section.

Selon un mode d'exécution, les margines sont récupérées soit en sortie d'un moulin à 3 phases (en sortie du décanteur centrifuge), soit d'un moulin à deux phases (en sortie de la centrifugeuse). Ces margines sont ensuite filtrées et utilisées telle quelle, ou extraites avant filtration avec un solvant organique polaire tel que : alcools en C1-C4, dont le méthanol et l'éthanol, préférentiellement l'éthanol. L'étape d'extraction peut se faire sous assistance ultra-sons, ou après une étape de concentration. La teneur en solvant organique polaire pour l'extraction des margines se situe de 5 à 50% v/v, préférentiellement à 20% (valeur minimale assurant une stabilité microbiologique). Cette étape d'extraction est suivie par une étape de filtration sur filtre AF15, avec ou sans adjuvant de filtration de type spindalite, afin d'obtenir un extrait fluide limpide.

Les margines contiennent également des tanins (jusqu'à 12g/l), dont certains responsables de la coloration noire (après oxydation survenue lors du broyage du fruit), et des protéines (1.2 à 2.4%). Une étape d'ultra-filtration supplémentaire avec un seuil de coupure compris entre 5 et 20 kDa permet donc l'élimination de ces macromolécules et une décoloration de l'extrait.

L'extrait de margines, décoloré par ultra-filtration ou non, est ensuite utilisé tel quel (avec le solvant d'extraction) ou concentré à 70° brix ou mis à sec, ou encore mis sur un support composé de glycérine, propylène glycol, 1,3-propane diol, maltodextrine, lactose et/ou tout autre support hydrosoluble cosmétiquement acceptable. L'ajout d'un conservateur est également possible s'il s'avère nécessaire.

Selon un mode de mise en oeuvre particulier de l'invention, l'excipient cosmétiquement acceptable comprend un mélange eau/propane-diol.

Selon un autre mode de mise en oeuvre particulier de l'invention, l'excipient cosmétiquement acceptable comprend un mélange eau/propane-diol 50/50.

Selon un mode particulier de mise en oeuvre de l'invention, la composition comprend une quantité d'extrait comprise entre 0,05% et 10% en poids par rapport au poids total de la composition.

La teneur en acides phénoliques exprimée en tyrosol mesurée par la technique de Folin des extraits obtenus est comprise entre 0.1 et 30% (m/m), préférentiellement entre 0.2 et 10% (m/m).

Avec le temps, le cuir chevelu devient sec et inconfortable. Les cheveux s'affinent, se dévitalisent, perdent de la matière.

La composition capillaire, selon l'invention, aux margines d'Olivier, est destinée à régénérer, redensifier et vitaliser les cheveux en perte de matière, et plus particulièrement les cheveux affaiblis par le temps.

Elle redonne de la consistance aux cheveux des racines jusqu'aux pointes.

La composition des margines d'olivier, est un soin capillaire anti-âge qui apporte les éléments essentiels permettant au cuir chevelu de puiser une nouvelle source de jeunesse et aux cheveux de retrouver densité, matière et vitalité.

Selon un mode d'exécution, la composition selon l'invention est présentée sous une forme adaptée à une administration topique.

Les excipients acceptables au sens de la présente invention permettent en particulier d'assurer une bonne stabilité, une texture et un toucher agréables. Il peut aussi s'agir par exemple d'agents de formulations ou d'additifs d'usage connu et classique en cosmétique : on peut citer des tensioactifs, colorants, conservateurs, parfums, etc.

Les compositions selon l'invention peuvent se présenter sous les formes qui sont habituellement connues pour une administration topique sur les cheveux, c'est-à-dire notamment un shampoing, un après shampoing, une crème capillaire, une lotion capillaire ou un spray sans rinçage.

Dans un mode de réalisation particulier, il a été mis en évidence une supériorité d'action de l'extrait associé à des supports hydro-glycérinés ou hydroglycoliques par rapport au véhicule seul mais aussi par rapport à l'extrait seul simplement dilué dans l'eau, montrant l'activité propre de l'extrait, et une potentialisation de son activité en présence de ces solvants. Ces solvants seront donc préférentiellement utilisés comme support de l'extrait, ou présent dans la composition cosmétique. Il a été également démontré que malgré l'étape de purification par ultra-filtration, l'activité est conservée.

Un autre objet de la présente invention concerne un procédé de soin cosmétique des cheveux destiné à améliorer l'esthétique des cheveux en leur redonnant densité, matière et vitalité caractérisé en ce qu'il consiste à appliquer sur les cheveux une composition cosmétique comprenant une quantité efficace d'un extrait de margines d'olivier, à laisser celle-ci au contact des cheveux, et éventuellement à rincer les cheveux.

L'invention sera mieux comprise à la lecture des résultats ci-dessous qui l'illustrent sans en limiter la portée.

### Exemples d'extraits

### Exemple 1 :

80 L de margines sont stabilisées avec 20.66 L d'Ethanol 96°C de façon à obtenir un degré alcoolique de 20°, assurant une stabilité microbiologique de la matière première. Ensuite, cette solution est centrifugée et le surnageant est filtré 2 fois sur filtre K900 avec l'aide d'un adjuvant de filtration type spindalite. Après filtration, la solution limpide est séchée, permettant l'obtention de 5.3 kg d'extrait de fruit d'olivier sous forme d'une poudre marron.

Exemple 2 :

300 L de margines sont stabilisées avec 77.48 L d'Ethanol 96°C, puis centrifugées et filtrées 2 fois sur filtre K900 avec l'aide d'adjuvant de filtration. La solution limpide obtenue est ensuite décolorée par ultra-filtration sur membrane de seuil de coupure de 10 kDa, puis l'alcool est éliminé et l'extrait concentré de façon à obtenir 15 kg d'un concentrât à 70% de matière sèche, stable d'un point de vu microbiologique et organoleptique.

### Exemple 3 :

300 L de margines sont stabilisées avec 77.48 L d'Ethanol 96°C, puis centrifugées et filtrées 2 fois sur filtre K900 avec l'aide d'adjuvant de filtration. La solution limpide obtenue est ensuite décolorée par ultra-filtration sur membrane de seuil de coupure de 10 kDa, puis l'alcool est éliminé et l'extrait concentré et séché de façon à obtenir 10,5 kg d'un extrait sec sous forme de poudre beige.

### Exemple 4 :

300 L de margines sont stabilisées avec 77.48 L d'Ethanol 96°C, puis centrifugées et filtrées 2 fois sur filtre K900 avec l'aide d'adjuvant de filtration. La solution limpide obtenue est ensuite décolorée par ultra-filtration sur membrane de seuil de coupure de 10 kDa, puis l'alcool est éliminé et l'extrait séché sur un support 1,3-propane diol / eau 1 :1 de façon à obtenir 230 kg d'un extrait hydroglycolique stable d'un point de vu microbiologique et organoleptique.

### Exemple 5 :

100 L de margines d'olive sont centrifugées. Le surnageant est filtré 2 fois sur filtre K900 avec l'aide d'adjuvant de filtration. La solution limpide obtenue est lyophilisée de façon à obtenir 10kg d'extrait sec de margines d'olive sous forme d'une poudre marron foncé.

### Tests d'activité

Des cheveux fins ont été mesurés par un micromètre à laser avant et après l'application des extraits.

### Méthode

### CHEVEUX UTILISES

Cheveux vierge, bruns et fins avec un diamètre ≤ 60µm, obtenu du fournisseur Kerling International (Allemagne).

Les cheveux ont été conditionnés par sertissage à l'aide de 2 tuyaux en laiton gainé en plastique à l'intérieur. La longueur de cheveu analysable est 30mm.

### APPLICATION DES PRODUITS

Les cheveux ont été traités en les laissant tremper dans le produit pour une durée de 1 à 5 minutes, puis enlevés et laissés sécher sur une feuille de polyéthylène à l'air libre pendant 1 heure au minimum. Une seule application a été effectuée.

### MESURE DU DIAMETRE DU CHEVEU

L'appareillage utilisé était un Laser Scan Micrometer (LSM, Mitutoyu, Japon).

Pour chaque cheveu, 5 mesures de diamètre ont été réalisées à des positions fixes sur une longueur de 10 mm. Ces mesures ont été réalisées avant et après l'application du produit.

Le paramètre de la section elliptique du cheveu en µm² a été calculé à partir des diamètres minimaux et maximaux, et utilisé pour indiquer les changements d'épaisseur lors des traitements.
Pendant ces mesures, la température et l'hygrométrie ambiante ont été relevées.

### Résultats

### Première étude :

Tableau 1 : Evolution du paramètre de section en µm² des cheveux avant et après l'application de :
- l'extrait tel que décrit dans l'exemple 1 à 5%, dilué dans un support eau / 1,3-propane diol 50 :50,
- l'extrait tel que décrit dans l'exemple 3 à 5% dilué dans un support eau / 1,3-propane diol 50 :50,
- et l'excipient seul (mélange eau / 1,3-propane diol 50 :50). (n=10, moyennes ± écart type).

**TABLEAU I**

| | Avant application | | Après application | | % Evolution | T-test apparié |
|---|---|---|---|---|---|---|
| Traitement | Moyenne | Ecart type | Moyenne | Ecart type | | Valeur p |
| 5% Ext selon ex.1 | 1801.6 | ± 463.98 | 1882.1 | ± 467.62 | 4.67 | < 0.0001 |
| 5% Ext selon ex. 3 | 1690.6 | ± 552.78 | 1743.2 | ± 576.77 | 3.03 | 0.0177 |
| Excipient seul | 1792.1 | ± 431.72 | 1820.5 | ± 460.14 | 1.35 | 0.0976 |

Pendant les 3 jours de mesures, les valeurs des conditions ambiantes relevées ont été :
> Température : 17,3 - 19,1 °C, moyenne 18,5°C ± 0,5
> Hygrométrie : 37% - 39%, moyenne 38,1% ± 1,8

### Deuxième étude :

Tableau 2 : Evolution du paramètre de section en µm² des cheveux avant et après application de :
- l'extrait tel que préparé dans l'exemple 1 dilué dans un support eau / glycérine 50 :50 (n=25, moyennes ± écart type),
- et 2) l'excipient seul (50/50 H₂0/ Glycérine). (n=5, moyennes ± écart type).

**TABLEAU II**

| | Avant application | | Après application | | % Evolution | T-test apparié |
|---|---|---|---|---|---|---|
| Traitement | Moyenne | Ecart type | Moyenne | Ecart type | | Valeur p |
| 5% Ext selon ex. 1 | 1857.0 | ± 483.0 | 2160.9 | ± 597.0 | 16.51 | < 0.0001 |
| Excipient seul | 1965.6 | ± 458.0 | 1993.1 | ± 421.5 | 1.77 | 0.558 |

Pendant les 2 jours de mesures, les valeurs des conditions ambiantes relevées ont été :
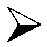 Température : 22,0 - 24,0 °C, moyenne 23,2°C ± 0,6
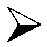 Hygrométrie : 53% - 64%, moyenne 57,2% ± 3,4

### Conclusion

Une augmentation de l'épaisseur du cheveu se traduit par une augmentation de sa section.

Lors de la première étude, on observe une augmentation significative de la section pour les extraits préparés selon l'exemple 1 et selon l'exemple 3. Pour l'excipient seul on n'observe pas d'effet significatif sur la section. Ainsi, les extraits préparés selon l'exemple 1 ou 3 apportent une action significative d'épaississement de la tige du cheveu.

Lors de la seconde étude, on observe une augmentation hautement significative de la section pour l'extrait préparé selon l'exemple 1 dilué dans le mélange eau/glycérine. Une fois encore, nous avons démontré que l'excipient seul n'entraîne pas d'effet significatif sur la section. Ceci nous permet de confirmer dans cette seconde étude que l'extrait préparé selon l'exemple 1 apporte une action significative d'épaississement de la tige du cheveu.

A titre d'illustration de la présente invention, on indiquera ci-après un exemple détaillé de composition cosmétique selon l'invention qui, de façon générale, peut se présenter soit sous la forme d'un spray sans rinçage contenant de 0.1% à 10% d'un extrait de type de celui obtenu à l'exemple, soit sous la forme d'un shampoing de 0.05% à 10% d'un tel extrait de margines d'olivier.

### Exemple de composition capillaire :

| | |
|---|---|
| Extrait margines olivier selon ex 2 | 0.5 à 3% |
| SODIUM LAURETH SULFATE | 9 à 11% |
| ALKYLETHER SULFATE Na Mg | 2 à 4 % |
| SODIUM COCOAMPHOACETATE | 1 à 2% |
| TRISODIUM ETHYLENEDIAMINE DISUCCINATE | 0.1 à 0.3% |
| COCO-GLUCOSIDE | 0.2 à 1% |
| HESPERIDIN METHYL CHALCONE | 0.1 à 0.5% |
| TOCOPHERYL NICOTINATE | 0.1 à 0.3% |
| BETAINE | 0.5 à 2% |
| GLYCERIN | 1 à 5% |
| HYDROXYPROPYL GUAR | |
| HYDROXYPROPYLTRIMONIUM CHLORIDE | 0.1 à 0.5% |
| propane | 1,3 diol qsp |
| Colorants | qsp |
| Acide citrique | qsp |
| Conservateurs | qsp |
| Eau | qsp |

## Revendications

1. Utilisation d'une composition capillaire comprenant à titre de principe actif un extrait de margines d'olivier et comprenant en outre au moins un excipient cosmétiquement acceptable, pour redensifier le cheveu par une augmentation de l'épaisseur du cheveu.

2. Utilisation selon la revendication 1 **caractérisée en ce que** la section du cheveu est augmentée.

3. Utilisation selon les revendications 1 ou 2 **caractérisée en ce que** la composition est présentée sous une forme appropriée à une application topique.

4. Utilisation selon l'une quelconque des revendications 1 à 3 **caractérisée en ce que** la composition se présente sous forme de shampoing, après shampoing, crème capillaire, lotion capillaire ou spray sans rinçage.

5. Utilisation selon l'une quelconque des revendications 1 à 4 **caractérisée en ce que** l'extrait de margines d'olivier est obtenu par une extraction avec un solvant organique polaire choisi parmi les alcools en C1-C4, préférentiellement l'éthanol.

6. Utilisation selon l'une quelconque des revendications 1 à 5 **caractérisée en ce que** l'excipient cosmétiquement acceptable comprend un produit choisi dans le groupe constitué par la glycérine, le propylène glycol, le 1,3-propane diol, la maltodextrine et le lactose, et tout autre support hydrosoluble cosmétiquement acceptable.

7. Utilisation selon l'une des revendications 1 à 6, **caractérisée en ce que** l'excipient cosmétiquement acceptable comprend un mélange eau/propane-diol.

8. Utilisation selon la revendication 7, **caractérisée en ce que** l'excipient cosmétiquement acceptable comprend un mélange eau/propane-diol 50/50.

9. Utilisation selon l'une des revendications 1 à 8, **caractérisée en ce que** l'extrait de margines d'olivier présente une teneur en acides phénoliques exprimée en tyrosol, comprise entre 0,1 et 30 % (m/m), de préférence entre 0,2 et 10 % (m/m).

10. Utilisation selon l'une quelconque des revendications 1 à 9 **caractérisée en ce que** la composition comprend une quantité d'extrait comprise entre 0,05% et 10% en poids par rapport au poids total de la composition.

## Patentansprüche

1. Verwendung einer Zusammenssetzung für die Haarbehandlung, die als Wirkstoff einen Extrakt aus Olivenbaum-Fruchtwasser und ferner zumindest einen kosmetisch akzeptablen Trägerstoff enthält, um das Haar durch eine Verdickung des Haares zu verdichten.

2. Verwendung nach Anspruch 1, **dadurch gekennzeichnet, dass** der Querschnitt des Haares vergrößert ist.

3. Verwendung nach Anspruch 1 oder 2, **dadurch gekennzeichnet, dass** die Zusammensetzung in einer für die topische Anwendung geeigneten Form vorliegt.

4. Verwendung nach einem der Ansprüche 1 bis 3, **dadurch gekennzeichnet, dass** die Zusammensetzung in Form von Shampoo, Apres-Shampoo, Haarcreme, Haarwasser oder Spray ohne Spülung vorliegt.

5. Verwendung nach einem der Ansprüche 1 bis 4, **dadurch gekennzeichnet, dass** der Olivenbaum-Fruchtwasserextrakt durch Extraktion mit einem polaren organischen Lösungsmittel gewonnen wird, das aus C1-C4-Alkoholen, vorzugsweise Ethanol, gewonnen wird.

6. Verwendung nach einem der Ansprüche 1 bis 5, **dadurch gekennzeichnet, dass** der kosmetisch akzeptable Hilfsstoff ein Produkt enthält, das aus der Gruppe bestehend aus Glycerin, Propylenglykol, 1,3-Propandiol, Maltodextrin und Laktose und allen anderen kosmetisch akzeptablen wasserlöslichen Trägern ausgewählt ist.

7. Verwendung nach einem der Ansprüche 1 bis 6, **dadurch gekennzeichnet, dass** der kosmetisch akzeptable Träger ein Wasser/Propandiol-Gemisch enthält.

8. Verwendung nach Anspruch 7, **dadurch gekennzeichnet, dass** der kosmetisch akzeptable Hilfsstoff eine 50/50-Wasser/Propandiol-Mischung umfasst.

9. Verwendung nach einem der Ansprüche 1 bis 8, **dadurch gekennzeichnet, dass** der Olivenbaum-Fruchtwasserextrakt einen Phenolsäuregehalt, ausgedrückt als Tyrosol, zwischen 0,1 und 30 % (m/m), vorzugsweise zwischen 0,2 und 10 % (m/m), aufweist.

10. Verwendung nach einem der Ansprüche 1 bis 9, **dadurch gekennzeichnet, dass** die Zusammensetzung eine Menge an Extrakt umfasst, die zwischen 0,05 Gew.-% und 10 Gew.-%, bezogen auf das Gesamtgewicht der Zusammensetzung beträgt.

## Claims

1. Use of a hair-care composition comprising as active principle an extract of olive-tree vegetable water and further comprising at least one cosmetically acceptable excipient, to increase the density of hair by increasing the thickness of the hair.

2. Use according to claim 1 **characterized in that** the section of the hair is increased.

3. Use according to claims 1 or 2 **characterized in that** the composition is in a form suitable for topical application.

4. Use according to any one of claims 1 to 3, **characterized in that** the composition is in the form of shampoo, conditioner, hair cream, hair lotion or leave-in spray.

5. Use according to any one of claims 1 to 4, **characterized in that** the extract of olive-tree vegetable water is obtained by extraction with a polar organic solvent selected from C1-C4 alcohols, preferably ethanol.

6. Use according to any one of claims 1 to 5 **characterized in that** the cosmetically acceptable excipient comprises a product selected from the group consisting of glycerine, propylene glycol, 1,3-propane diol, maltodextrin and lactose, and any other cosmetically acceptable water-soluble carrier.

7. Use according to any one of claims 1 to 6, **characterized in that** the cosmetically acceptable excipient comprises a water/propanediol mixture.

8. Use according to claim 7, **characterized in that** the cosmetically acceptable excipient comprises a 50/50 water/propanediol mixture.

9. Use according to any one of claims 1 to 8, **characterized in that** the extract of olive-tree vegetable water has a phenolic acid content expressed as tyrosol of between 0.1 and 30 % (m/m), preferably between 0.2 and 10 % (m/m).

10. Use according to any one of claims 1 to 9 **characterized in that** the composition comprises an amount of extract between 0.05 % and 10 % by weight based on the total weight of the composition.
